# EUROPÄISCHE PATENTSCHRIFT

(11) **EP 1 150 734 B1**
(45) Veröffentlichungstag und Bekanntmachung des Hinweises auf die Patenterteilung: **20.10.2004**
(21) Anmeldenummer: 00905057.6
(22) Anmeldetag: 11.02.2000
(51) Int. Cl.: A61M 16/04

(54) **VORRICHTUNG ZUM EINFÜHREN EINES INTUBATIONSTUBUS IN DIE TRACHEA**
DEVICE FOR INTRODUCING AN INTUBATION TUBE INTO THE TRACHEA
DISPOSITIF D'INTRODUCTION D'UN TUBE D'INTUBATION DANS LA TRACHEE

(30) Priorität: 12.02.1999 DE 19905840; 24.08.1999 DE 19939971
(43) Veröffentlichungstag der Anmeldung: 07.11.2001
(73) Patentinhaber: Karl Storz GmbH & Co., 78532 Tuttlingen (DE)
(72) Erfinder: BERCI, George, Los Angeles, CA 90025 (US); LIPP, Markus, D-55128 Mainz (DE); PRÄDEL, Christian, D-78532 Tuttlingen (DE)
(74) Vertreter: Weller, Wolfgang, Dr.
(86) Internationale Anmeldenummer: PCT/EP2000/001138
(87) Internationale Veröffentlichungsnummer: WO 2000/047263

(56) Entgegenhaltungen:
- EP-A- 0 664 101
- WO-A-91/12044
- DE-C- 4 137 426
- US-A- 3 822 697
- US-A- 4 254 762
- US-A- 5 740 791

## Beschreibung

Die Erfindung betrifft eine Vorrichtung zum Einführen eines Intubationstubus in die Trachea, mit einem Schaft, mit einer am distalen Ende des Schaftes angeordneten Aufnahme, an die ein proximales Ende eines Intubationstubus anbringbar ist, und mit einer am proximalen Ende des Schafts angeordneten Kupplung zum Ankuppeln eines Endoskopes.

Eine derartige Vorrichtung ist aus der WO-A-91 12044 bekannt.

Aus der US-A-5 740 791 ist ein teleskopartiger Schaft für eine orale Spreizvorrichtung bekannt, um die Atemwege für Tuben und andere Geräte freizuhalten. Die Länge des Schaftes wird bei der Intubation nicht verändert.

Die Einführung eines Intubationstubus in die Trachea ist ein äußerst schwierig durchzuführender Vorgang, der insbesondere bei Kindern und im Falle von Mißbildungen nur sehr schwierig durchzuführen ist. Ein Intubationstubus besteht meist in Form eines Schlauches aus Kunststoffmaterialien, der, nachdem er in die Luftröhre eingeführt wird, dort mit Hilfe eines aufblasbaren Ballons am Ende gesichert wird, um das freibleibende Volumen der Trachea abzuschließen. Dazu muß der Tubus unter der Epiglottis und durch die Stimmbänder hindurchgeführt werden. Dieser Bereich ist nur eingeschränkt von außen her ersichtlich.

Insbesondere bei Kleinkindern ist dieser Vorgang äußerst schwierig durchzuführen und muß mit größter Vorsicht durchgeführt werden, um die vorgenannten Körperteile nicht zu verletzen.

Insbesondere bei Notfällen muß der Tubus ggf. sehr rasch gesetzt werden, um eine alsbaldige Sauerstoffversorgung sicherzustellen. Bei Notfällen, bspw. bei Verätzungen oder Vergiftungen von Kleinkindern, ist die Intubation noch bei Bewußtsein des Kindes durchzuführen, das sich dabei üblicherweise stark wehrt.

Es besteht daher ein Bedarf an einer Vorrichtung, mit der ein solcher Tubus rasch, zielgerecht und sicher in die Trachea eingeführt werden kann. Dabei besteht außerdem der Wunsch, das distale Ende des Tubus während des Einführvorganges andauernd im Blickfeld zu haben bzw. das Vorschieben dieses Endes visuell beobachten zu können.

Es ist daher Aufgabe der vorliegenden Erfindung, eine entsprechende Vorrichtung zum Einführen eines Intubationstubus in die Trachea zu schaffen.

Erfindungsgemäß wird die Aufgabe dadurch gelöst, daß die Länge des Schaftes veränderbar ist.

Durch das Vorsehen einer Aufnahme am distalen Ende des Schaftes kann der Intubationstubus sicher und festsitzend an die Vorrichtung angebracht werden. Dieser Vorgang kann vor dem eigentlichen Eingriff schon vorbereitet werden, d.h. abhängig von dem Alter und der Größe des Patienten kann ein entsprechender geeigneter Intubationstubus an die Vorrichtung angebracht werden. Übliche Längen von Intubationstuben reichen von etwa 12 cm bei Kleinkindern bis etwa 35 cm bei Erwachsenen. Das Vorsehen einer Kupplung zum Ankoppeln eines Endoskopes am proximalen Ende erlaubt es nun, direkt an die Vorrichtung ein optisches Instrument anzubringen, über das der Einführvorgang dauernd visuell beobachtet werden kann. Es kann vorgesehen sein, das Endoskop so anzukoppeln, daß dieses integraler Bestandteil der Vorrichtung ist und daher auch fest und unlösbar angekoppelt sein kann.

In diesem Fall sind dann die Länge der Vorrichtung, die Länge des Intubationstubus und die Länge des Endoskopschaftes so aufeinander abzustimmen, daß das distale Ende des Endoskopes gerade im Bereich des distalen Endes des Intubationstubus zum Liegen kommt. Sowohl der Intubationstubus als auch das Endoskop sind festsitzend jedoch lösbar an der Vorrichtung anbringbar, so daß der Zusammenbau aus diesen drei Bauelementen ein kompaktes und schlankes Gebilde darstellt, das vom Operateur einfach zu handhaben ist und diesem zugleich die Möglichkeit gibt, visuell das Einführen zu beobachten. Das Endoskop kann dabei starr, semi-flexibel oder auch flexibel sein.

Nach Setzen des Intubationstubus kann das Endoskop wieder abgekoppelt und abgenommen werden, und auch die Vorrichtung kann vom proximalen Ende des Intubationstubus gelöst werden, so daß dieser dann an die entsprechenden Beatmungsgeräte oder dgl. angeschlossen werden kann. In dieser Ausgestaltung ist die Vorrichtung insbesondere geeignet, bei operativen Vorgängen eingesetzt zu werden, bei denen zuvor optimale Längen und Größen von Intubationstubus und Endoskop bereitgestellt werden können.

Über die Längenveränderung des Schaftes kann mit ein und derselben Vorrichtung an die jeweils vorhandenen Gegebenenheiten angepaßt werden. Das bedeutet, durch die Längenveränderung des Schaftes kann das angekoppelte Endoskop so hin- und herverschoben werden, daß jeweils der gewünschte Sichtbereich erhalten wird, also insbesondere das Endoskop im Bereich des distalen Endes des Beatmungsschlauches zum Liegen kommt, und zwar unabhängig davon, wie lang der Intubationstubus ist. Anders ausgedrückt, kann eine einzige derartige Vorrichtung mit einem längenveränderbaren Schaft für unterschiedliche Längen an Intubationstuben eingesetzt werden, so daß nicht mehrere, auf eine ganz bestimmte Länge eines Intubationstubus bzw. ganz bestimmte Endoskope abgestimmte Vorrichtungen bereitgestellt werden müssen.

Dies ist insbesondere auch in Notfällen hilfreich, bei denen bspw. nur gerade ein bestimmter Intubationstubus vorliegt und entsprechend auch nur ein bestimmtes Endoskop, so daß durch Längenveränderung des Schaftes dann die jeweils optimale Beobachtungsposition für den Einführvorgang durchgeführt werden kann.

Bei anatomischen Anomalien kann dann durch Längenveränderung des Schaftes das Endoskop hin- und herbewegt werden, um jeweils optimale Sichtbedingungen zu schaffen, so daß auch bei solchen Anomalien rasch und atraumatisch der Intubationstubus in die Trachea eingeführt werden kann.

In einer weiteren Ausgestaltung der Erfindung ist der Schaft teleskopartig aufgebaut.

Diese Ausgestaltung hat den Vorteil, daß der Schaft trotz der Längenveränderbarkeit ein schlankes Bauelement darstellt und daß der Stauraum der Vorrichtung relativ gering ist. Bei Nichtgebrauch kann der Schaft teleskopartig zusammengeschoben werden.

In einer weiteren Ausgestaltung ist der Schaft rohrförmig.

Diese Maßnahme hat den Vorteil, daß der Schaft des Endoskopes durch den Schaft hindurchgeschoben werden kann und allseits von diesem umgeben und somit geschützt ist. Die rohrförmige Ausbildung des Schaftes ermöglicht dann auch, weitere Instrumente oder auch ggf. andere Medien wie Gase oder Flüssigkeiten durch diesen hindurchzuführen.

In einer weiteren Ausgestaltung der Erfindung ist der Schaft aus mehreren teleskopartig ineinanderverschiebbaren Rohren aufgebaut.

Diese Maßnahme vereinigt auf konstruktiv einfache Art und Weise zum einen die Längenveränderbarkeit des Schaftes und zugleich die Ausbildung als Rohr zum Durchführen oder zum Durchleiten von Instrumenten oder Medien.

In einer weiteren Ausgestaltung der Erfindung ist am proximalen Ende der Vorrichtung ein Gasanschluß angeordnet, der mit dem Innenraum des hohlen Schaftes verbunden ist.

Diese Maßnahme hat den erheblichen Vorteil, daß schon beim Setzen des Intubationstubus lebensnotwendiger Sauerstoff zugeführt werden kann, was insbesondere bei Notfällen lebensentscheidend sein kann. Dadurch ist der Operateur bei Notfällen, in denen eine Erstickungsgefahr droht, schon beim Zuführen des Intubationstubus in der Lage, auch gleichzeitig Sauerstoff zuzuführen und muß nicht abwarten, bis nach Setzen des Intubationstubus ein Sauerstoffanschluß bewerkstelligt wird.

In einer weiteren Ausgestaltung der Erfindung ist am distalen Ende der Vorrichtung eine Handhabe angeordnet, über die die Vorrichtung handhabbar ist.

Diese Maßnahme hat den erheblichen Vorteil, daß der Operateur die Vorrichtung beim Einführen des Intubationstubus über die Handhabe fest und sicher in einer Hand halten und führen kann, so daß ihm die andere Hand für andere Manipulationen freisteht.

In einer weiteren Ausgestaltung der Erfindung ist die Aufnahme am distalen Ende des Schaftes der Vorrichtung zum Anbringen des Intubationstubus als Zapfen ausgebildet, auf den ein Ende des Intubationstubus aufschiebbar ist.

Diese Maßnahme hat den Vorteil, daß durch einen einfachen Aufschiebevorgang der Intubationstubus an die Vorrichtung angesetzt werden kann und dementsprechend, nach Setzen des Intubationstubus, auch dieser Zusammenbau wieder gelöst werden kann.

In einer weiteren Ausgestaltung der Vorrichtung ist die Aufnahme als Hohlzapfen ausgebildet, in den ein proximales Ende des Intubationstubus einschiebbar ist.

Diese Maßnahme hat den Vorteil, daß durch das Einführen des Intubationstubus in den Hohlzapfen eine radial schlanke Bauweise erzielt werden kann.

In einer weiteren Ausgestaltung der Erfindung ist der Zapfen leicht konisch ausgebildet.

Diese Maßnahme hat den Vorteil, daß der Intubationstubus fest haftend, jedoch nach wie vor lösbar auf der Aufnahme sitzt und daß durch die konischen aneinanderliegenden Flächen eine ausreichende Dichtfläche vorhanden ist, um ein Entweichen von Gas, das durch die Vorrichtung und den Intubationstubus hindurchgeführt wird, zu verhindern.

In einer weiteren Ausgestaltung der Erfindung ist der Schaft derart ausgebildet, daß ein Schaft des Endoskopes von proximal durch die Vorrichtung hindurchschiebbar ist.

Diese Maßnahme hat den Vorteil, daß durch einen einfachen Einschiebevorgang das Endoskop in die Vorrichtung eingeführt wird, der empfindliche Endoskopschaft vom Schaft der Vorrichtung umgeben und geschützt ist, so daß letztendlich vom proximalen Ende der Vorrichtung lediglich der an dieses angekoppelte Optikkopf des Endoskopes vorsteht. Dabei werden vorzugsweise semi-flexible Endoskope eingesetzt, d.h. deren Endoskopschaft ist in der Lage, den Krümmungen, die ein Intubationstubus beim Einführen in die Trachea einnimmt, zu folgen.

In einer weiteren Ausgestaltung der Erfindung ist der Schaft derart ausgebildet, daß auch bei eingeschobenem Endoskop noch ein Gas durch den Schaft hindurchführbar ist.

Diese Maßnahme hat den Vorteil der Multifunktionalität dahingehend, daß trotz eingeschobenem Endoskop auch noch durch den Schaft ein Beatmungsgas hindurchgeführt werden kann.

In einer weiteren Ausgestaltung der Vorrichtung ist vorgesehen, daß diese über deren Länge mit einem Schlitz versehen ist, über den die Vorrichtung an einen Schaft eines Endoskopes anlegbar ist.

Diese Maßnahme ist dann von besonderem Vorteil, wenn die Vorrichtung an ein starres Endoskop angelegt werden soll, dessen Schaft am distalen Ende abgeknickt ist, ein sogenanntes Intubations-Fiberskop. Durch Vorsehen eines Schlitzes kann nun die Vorrichtung im geradlinig verlaufenden Bereich des Schaftes, also nach dem distalseitigen Knick des Schaftes, an diesen in einer radialen Bewegung angelegt und dann Richtung proximalem Ende verschoben und mit dem Endoskop verkoppelt werden. Es können dann Drehschieber vorgesehen sein, die nach Anlegen der Vorrichtung an den Schaft des Endoskopes über den Schlitz gedreht werden und so ein seitliches Abfallen der Vorrichtung sperren.

In einer weiteren Ausgestaltung ist vorgesehen, daß die Vorrichtung über die Kupplung an das Endoskopgehäuse anklemmbar ist.

Diese konstruktiv besonders einfache Ausgestaltung hat den Vorteil, daß die Vorrichtung an einem vorhandenen herkömmlichen Endoskop ohne eine besondere Anpassung des Endoskops im Bereich des Endoskopgehäuses befestigt werden kann. Eine Schraube kann zur Verbesserung der Klemmwirkung vorgesehen sein.

In einer weiteren Ausgestaltung der Erfindung ist die Kupplung als Rastmechanismus zum Verrasten der Vorrichtung mit dem Endoskop ausgebildet oder ist mittels einer Überwurfmutter-Gewinde-Verbindung an dem Endoskopgehäuse befestigbar.

Im Fall eines Rastmechanismus zum Verrasten der Vorrichtung am Endoskop ist weiterhin bevorzugt, wenn der Rastmechanismus zumindest eine Kugelraste aufweist, und wenn am Endoskopgehäuse zumindest eine Vertiefung ausgebildet ist, in die die Kugelraste eingreift.

Die Befestigung der Vorrichtung über eine in eine Vertiefung am Endoskop einrastende Kugelraste hat den besonderen Vorteil, daß die Vorrichtung schnell am Endoskop sicher haltend befestigt und eben so schnell wieder gelöst werden kann.

Dabei ist es weiterhin bevorzugt, wenn die Vertiefung als umfängliche Nut am Endoskop ausgebildet ist.

Diese Maßnahme hat den Vorteil, daß beim Anbringen der Vorrichtung an dem Endoskop nicht auf eine bestimmte Drehorientierung der Vorrichtung in bezug auf die Achse des Endoskopes geachtet werden muß, weil sich die Vorrichtung bei dieser Ausgestaltung in jeder Drehstellung um die Endoskoplängsachse an diesem befestigen läßt.

Als weitere Ausgestaltung ist vorgesehen, die Befestigung der Vorrichtung am Endoskopgehäuse durch eine Bajonettverriegelung auszugestalten.

In einer weiteren Ausgestaltung der Erfindung ist die Kupplung einstückig mit dem Endoskop ausgebildet.

Diese Maßnahme hat den Vorteil, daß die Vorrichtung fest mit dem Endoskop verkuppelt ist.

In einer weiteren Ausgestaltung der Erfindung ist das Endoskop mit einer Kamera versehen.

Diese Maßnahme hat den Vorteil, daß der Operateur mit beiden Augen das jeweilige Bild erfassen kann und die schwierige Anatomie und ggf. Anomalien erkennen und darauf reagieren kann, so daß der Tubus möglichst atraumatisch und zutreffend sitzend eingeführt werden kann.

Es versteht sich, daß die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung dargestellt und werden in der nachfolgenden Beschreibung näher erläutert. Es zeigen:
- Fig. 1: einen Längsschnitt eines ersten Ausführungsbeispiels einer erfindungsgemäßen Vorrichtung mit längenveränderbarem Schaft in Form von drei Teleskoprohren in vollständig ausgezogenem Zustand,
- Fig. 2: eine der Fig. 1 entsprechende Schnittdarstellung in völlig zusammengeschobenem Zustand der Vorrichtung, wobei ein Abschnitt eines Intubationstubus gezeigt ist, der auf eine Aufnahme aufschiebbar ist,
- Fig. 3: eine Seitenansicht der Vorrichtung von Fig. 2 in eingeschobenem Zustand mit proximaler Kupplung, wobei gerade ein Endoskop von proximal her in die Vorrichtung eingeschoben wird,
- Fig. 4: eine stirnseitige Ansicht der Vorrichtung von distal,
- Fig. 5: eine stirnseitige Ansicht der Vorrichtung von proximal,
- Fig. 6: einen Zusammenbau aus der Vorrichtung, einem Endoskop und einem Intubationstubus,
- Fig. 7: einen Längsschnitt eines weiteren Ausführungsbeispiels einer Vorrichtung, die über eine Feststellschraube mit einem Endoskop verkoppelt ist,
- Fig. 8: die Vorrichtung von Fig. 7 in einer weiteren teleskopartig ausgezogenen Betriebsstellung,
- Fig. 9: einen Längsschnitt eines weiteren Ausführungsbeispiels einer Vorrichtung mit einer Kupplung über eine Kugelraste, und
- Fig. 10: einen Längsschnitt eines weiteren Ausführungsbeispiels einer erfindungsgemäßen Vorrichtung, die einstückig mit einem Endoskop gekoppelt ist.

In den Zeichnungen ist eine erfindungsgemäße Vorrichtung in der Gesamtheit mit der Bezugsziffer 10 versehen.

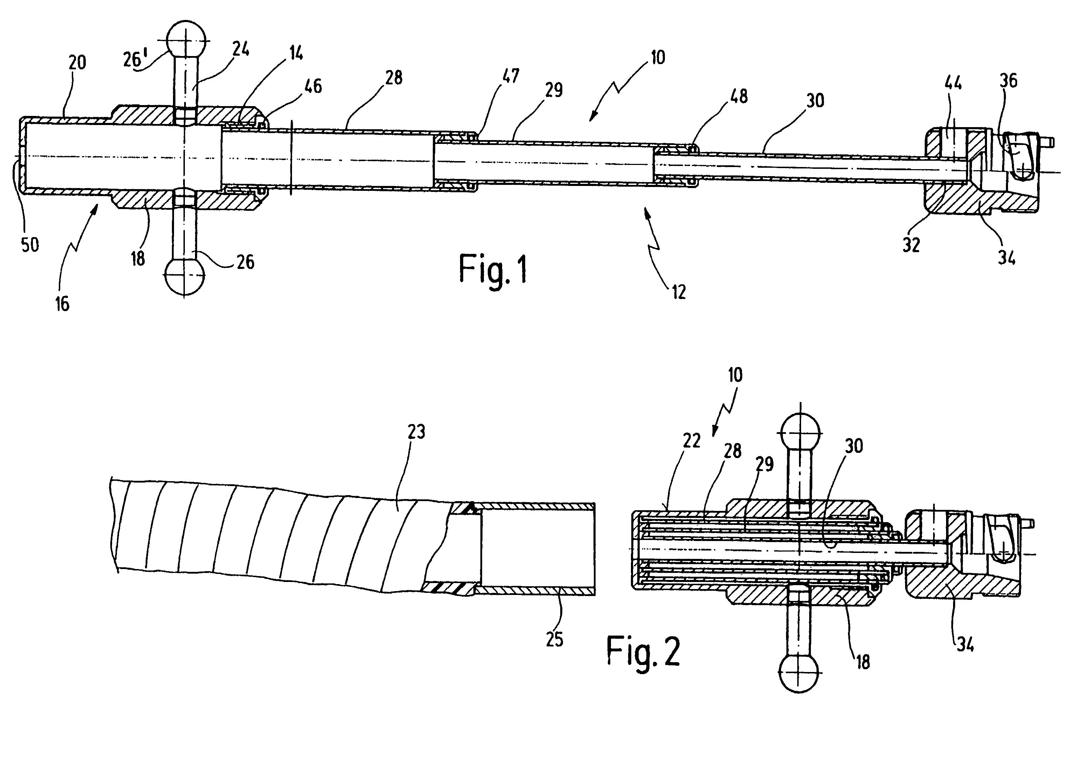

Aus der Schnittdarstellung von Fig. 1 ist ersichtlich, daß die Vorrichtung 10 einen mittigen zentralen Schaft 12 aufweist, an dessen distalem Ende 14 eine Aufnahme 16 angeordnet ist.

Die Aufnahme 16 weist ein etwa hohlrohrförmiges Gehäuse 18 auf, von dem distal ein hohlzylindrischer Zapfen 20 vorspringt. Die Außenseite 22 des Zapfens 20 dient dazu, wie das insbesondere aus der Darstellung von Fig. 2 ersichtlich ist, darauf ein proximales Endstück 25 eines Intubationstubus 23 passend aufzuschieben. Zapfen 20 und proximales Endstück 25 verlaufen leicht konisch, wodurch der Intubationstubus 23 festsitzend und dichtend auf den Zapfen 20 aufsetzbar ist.

Der Intubationstubus 23 besteht, wie an sich bekannt, aus einem Schlauch aus Kunststoffmaterial und weist eine Länge von etwa bis zu 12 cm zur Intubation von Kleinkindern, bis etwa 35 cm zur Intubation von Erwachsenen auf.

Wie aus den Fig. 1 und 2 ersichtlich, stehen von der Außenseite des Gehäuses 18 radial gegenüberliegend zwei Stäbe 26, 26' mit einem kugeligen Ende vor, wobei diese Stäbe 26, 26' als Handhabe 24 für die Vorrichtung 10 dienen.

Der Schaft 12 besteht aus drei teleskopartig ineinanderschiebbaren Rohren 28, 29 und 30.

Das Rohr 28 mit dem größten Durchmesser ist an seinem distalen Ende mit dem Gehäuse 18 gleitend jedoch unverlierbar verbunden.

In das Rohr 28 ist das Rohr 29 eingesetzt, in das wiederum das Rohr 30 eingesetzt ist.

O-Ringe 46, 47, 48 sorgen für eine gasdichte, jedoch teleskopartig ineinanderschiebbare Beweglichkeit der Rohre 28, 29 und 30 untereinander.

Es ist auch möglich, das Rohr mit dem kleinsten Durchmesser am Gehäuse anzubringen und auf dieses die anderen Rohre teleskopartig aufzuschieben.

Das proximale Ende des Schaftes 12, in der dargestellten Ausgestaltung also das proximale Ende des Rohres 30, ist mit einer Kupplung 34 verbunden.

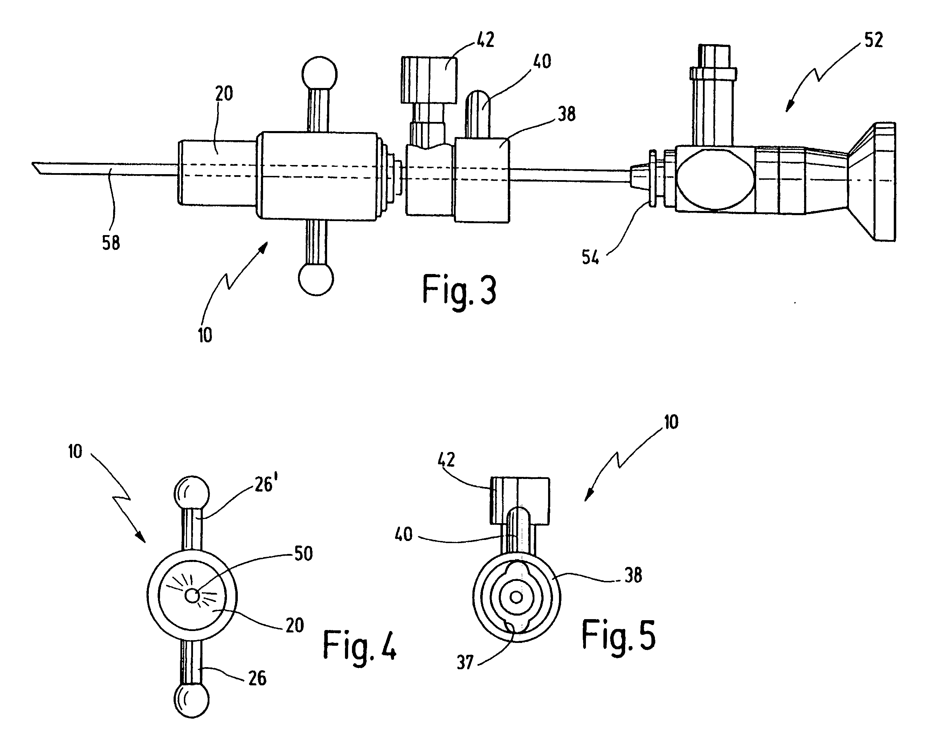

Die Kupplung 34 weist, wie das aus der Schnittdarstellung von Fig. 1 und 2 ersichtlich ist, eine Bajonettführung 36 auf. Diese Bajonettführung dient zur Führung eines in den Fig. 1 und 2 nicht dargestellten Verriegelungsringes 38, wie er aus den Fig. 3, 5 und 6 ersichtlich ist. Zum Verdrehen des Ringes 38 steht radial ein Stift 40 vor, wie das noch nachfolgend näher erläutert wird.

Aus der Schnittdarstellung von Fig. 1 ist zu erkennen, daß der Innenraum des Rohres 30 über einen seitlich abgehenden Stichkanal 44 mit der Außenseite verbunden ist. Auf diesem Stichkanal 44 ist ein Gasanschluß 42 montiert, wie er aus den Fig. 3, 5 und 6 ersichtlich ist. Es ist also möglich, ein Gas, bspw. Sauerstoff, über den Gasanschluß 42 von proximal nach distal durch die Vorrichtung 10 hindurchzuführen, wobei dann das Gas über eine Öffnung 50 am distalen Ende des Zapfens 20 austritt, bzw. bei auf diesen aufgeschobenem Intubationstubus 23 dann in den Intubationstubus 23 eingeführt wird.

Fig. 1 zeigt den Schaft 12 mit seiner Maximallänge, d.h. die drei Rohre 28, 29 und 30 sind zu einer Maximallänge teleskopartig auseinandergezogen.

Fig. 2 zeigt den Schaft 12 bzw. die Rohre 28, 29, 30 in maximal eingeschobenem Zustand.

Der Längenunterschied zwischen Fig. 1 und 2 gibt also das Maß der Längenveränderbarkeit des Schaftes 12 bzw. der Vorrichtung 10.

In Fig. 3 ist dargestellt, wie gerade ein Endoskop 52 von proximal her durch die Vorrichtung 10 hindurchgeschoben wird.

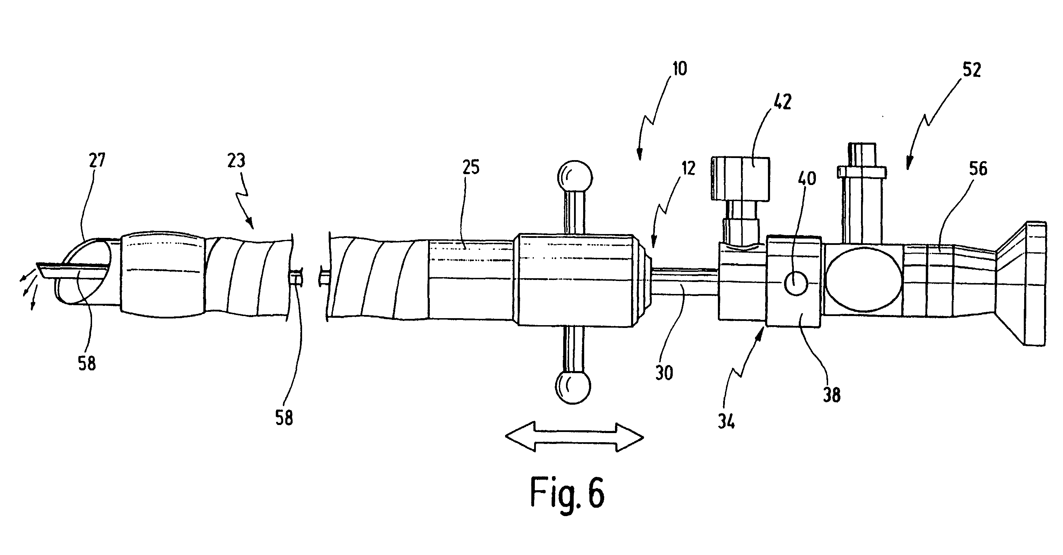

Das Endoskop 52 wird so weit vorgeschoben, bis dessen Kupplungsstück 54 durch die aus der Stirnansicht von Fig. 5 ersichtliche schlüssellochartige Öffnung 37 der Kupplung 34, die der Kontur des Kupplungsstückes 54 entspricht, hindurchgetreten ist. Durch Drehen des Verriegelungsringes 38 über den Stift 40 wird dann das vollständig eingeschobene Endoskop 52 mit der Kupplung 34 bzw. der Vorrichtung 10 verriegelt.

Dieser Zustand ist in Fig. 6 dargestellt, d.h. der Verriegelungsring 38 wurde gegenüber der Stellung von Fig. 3 etwa um 90° gedreht, um die Verriegelung zu bewerkstelligen. Aus Fig. 6 ist ferner ersichtlich, daß auf das distale Ende der Vorrichtung 10 ein Intubationstubus 23 aufgeschoben worden ist.

Die Länge des Schaftes 58 des Endoskopes 52 ist so gewählt, daß dessen distales Ende im Bereich des distalen Endes 27 des Intubationstubus 23 zum Liegen kommt. Dadurch ist es möglich, über das Endoskop 52 visuell die jeweilige Lage bzw. den Einführungszustand des distalen Endes 27 des Intubationstubus 23 zu beobachten. Durch Ein- und Ausfahren der teleskopartig ineinanderverschiebbaren Rohre 28, 29 und 30 kann nun das distale Ende des Endoskopes 52 in eine Relativstellung zum distalen Ende des Intubationstubus 23 gebracht werden, bei der jeweils optimale Sichtverhältnisse vorliegen.

Außerdem ist möglich, mit ein- und derselben Vorrichtung 10 unterschiedlich lange Intubationstuben einzusetzen, wobei dann bei einer bestimmten Endoskoplänge entsprechend die Länge des Schaftes durch Ein- bzw. Ausziehen der Rohre 28, 29 und 30 angepaßt wird. Bei Kleinkindern wird bspw. ein Endoskop mit einem Schaftdurchmesser von 2 mm und ein Tubus von 5 mm Außendurchmesser eingesetzt.

Zum Einführen eines Intubationstubus 23 wird der in Fig. 6 dargestellte Zusammenbau bewerkstelligt, also der Intubationstubus 23 auf den Zapfen 20 festsitzend aufgeschoben und ein Endoskop 52 angekoppelt. Dieser Zusammenbau wird unter der Epiglottis und durch die Stimmbänder geführt. Dabei wird die Zunge des Patienten über einen Laryngoskopspatel fixiert. Je nach Auswahl des Endoskops als starres, semi-flexibles oder flexibles Endoskop kann dessen Schaft einer Krümmung des Intubationstubus mehr oder weniger stark folgen. Bei einem semi-flexiblen Endoskop kann der Schaft um etwa ± 30° gebogen werden, je nach den anatomischen Gegebenheiten der Trachea. Es können bei bekannten Anomalien auch entsprechend gekrümmte Endoskope eingesetzt werden.

Anschließend wird der Tubus gelöst und in die Trachea vollständig eingeschoben, bis an die Luftröhrengabelung herangebracht und dort fixiert, wobei üblicherweise am distalen Ende des Intubationstubus 30 ein aufblasbarer Ballon vorhanden ist. Zum Lösen des Intubationstubus 23 können ein oder zwei Finger der Hand, die den Zusammenbau über die Handhabe 24 hält, das proximale Endstück 25 des Intubationstubus 23 von dem Zapfen 20 abschieben. Der Zusammenbau aus Vorrichtung 10 und Endoskop 52 kann nach wie vor zur Beobachtung herangezogen werden. Die Vorrichtung 10 wird dann samt Endoskop 52 wieder entfernt.

Die Vorrichtung 10 dient somit als Fixateur sowohl für den Intubationstubus als auch für die Beobachtungsoptik und stellt eine einfach und kompakt bauende Baueinheit zur Einführung eines Intubationstubus ein.

Soll schon beim Einführen Sauerstoff oder Beatmungsluft zugeführt werden, so kann dies über den seitlichen Gasanschluß bewerkstelligt werden.

Wie in Fig. 6 durch einen Doppelpfeil dargestellt, erlaubt die Längenveränderung des Schaftes 12 eine jeweils optimale Anpassung an die jeweilige Situation des Patienten, so daß auch bei schwierig zugänglichen Luftröhren, bspw. bei Mißbildungen oder bei Kleinkindern, ein rasches, sicheres und atraumatisches Einführen ermöglicht ist.

Soll anstatt einer direkten visuellen Beobachtung durch das Endoskop ein Bild über Videomonitor sichtbar gemacht werden, kann zusätzlich eine Kamera angesetzt werden, oder das Endoskop kann schon mit einer integrierten Kamera versehen sein, also schon als Videoendoskop ausgebildet sein. Durch Vergrößerung des Videobildes können anatomische Anomalien genau erkannt werden. Insbesondere bei der Intubation von Neugeborenen oder Säuglingen ist das besonders hilfreich. Der Operateur sieht das ggf. vergrößerte Videobild in seiner Arbeitsrichtung, so daß dieser die schwierige Anatomie und ggf. Anomalien deutlich erkennen kann und den Intubationstubus trotz dieser Schwierigkeiten zutreffend einführen kann.

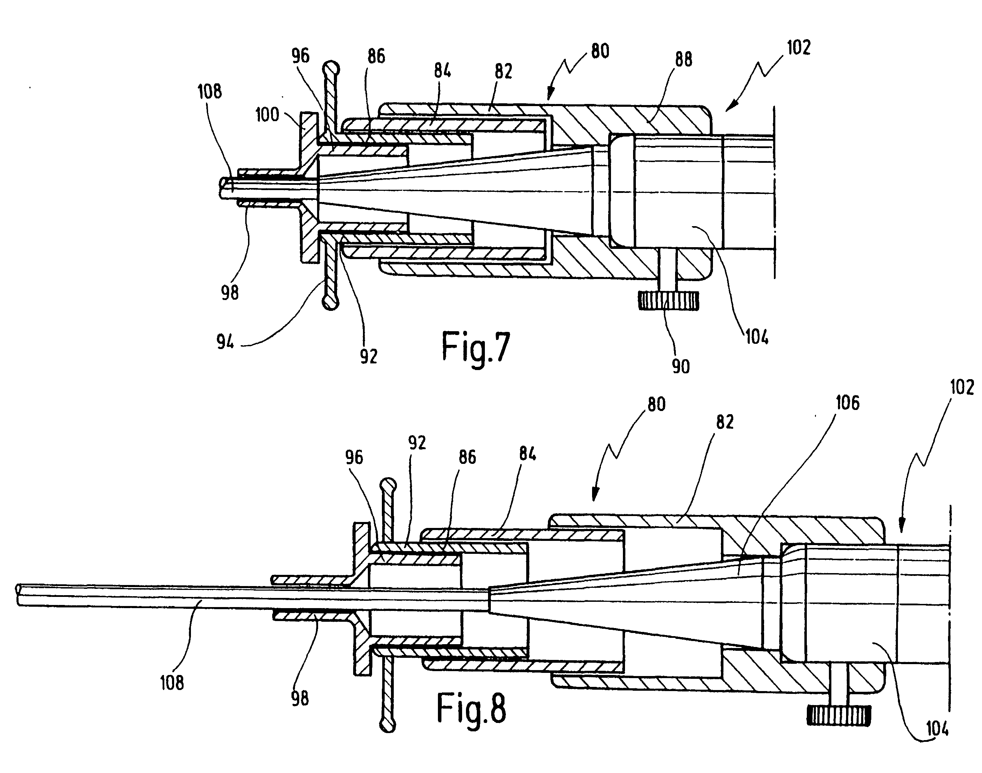

Die Fig. 7 und 8 zeigen ein weiteres Ausführungsbeispiel einer Vorrichtung 80, die an ein Endoskop 102 angekoppelt ist. Das Endoskop 102 ist ein flexibles Endoskop. Auch die Vorrichtung 80 ist längenveränderbar ausgebildet und weist dazu distalseitig einen Rohrfortsatz 82 auf, in den ein Rohrstück 84 eingeschoben ist. In dieses Rohrstück 84 ist ein weiteres Rohrstück 86 eingeschoben.

In Fig. 7 sind diese drei Rohrstücke teleskopartig ineinander eingeschoben dargestellt.

Eine Kupplung 88 zum Verbinden mit dem Endoskop 102 ist als proximalseitig vorstehendes Rohrstück ausgebildet, das auf einen entsprechenden Abschnitt des Optikkopfes 104 des Endoskops 102 aufschiebbar ist. Über eine radiale Feststellschraube 90 kann die Vorrichtung 80 am Endoskop 102 fixiert werden.

Der Optikkopf 104 des Endoskops 102 erstreckt sich über einen konisch verjüngenden Abschnitt 106 zu einem langerstreckten Schaft 108. Der konisch verjüngende Abschnitt 106 reicht durch den Innenraum der ineinandergeschobenen Rohre 84 und 86 hindurch. Eine Innenfläche 92 des innersten Rohres 96 dient als Aufnahme für einen Intubationstubus 98, und zwar für dessen proximales Ende 96.

Der Außendurchmesser des proximalen Endes 96 ist dabei so gewählt, daß er passend in die Aufnahme 92 eingeschoben werden kann. Für einen festen sicheren Sitz können Aufnahme 92 und proximaler Endabschnitt 96 konisch verlaufen.

Ein Ringflansch 100 bildet einen Anschlag für die Tiefe des Einschiebens des Intubationstubus 98 in die Aufnahme 92.

Vom innersten Rohr 86 steht radial eine Handhabe 94 vor, über die nicht nur die Vorrichtung 80 beim Koppeln mit dem Endoskop 102 gehandhabt werden kann, sondern diese Handhabe 94 dient auch dazu, die teleskopartig ineinandergeschobenen Rohre 84 und 86 zu verschieben.

Ein solcher längsverschobener Zustand ist in Fig. 8 dargestellt.

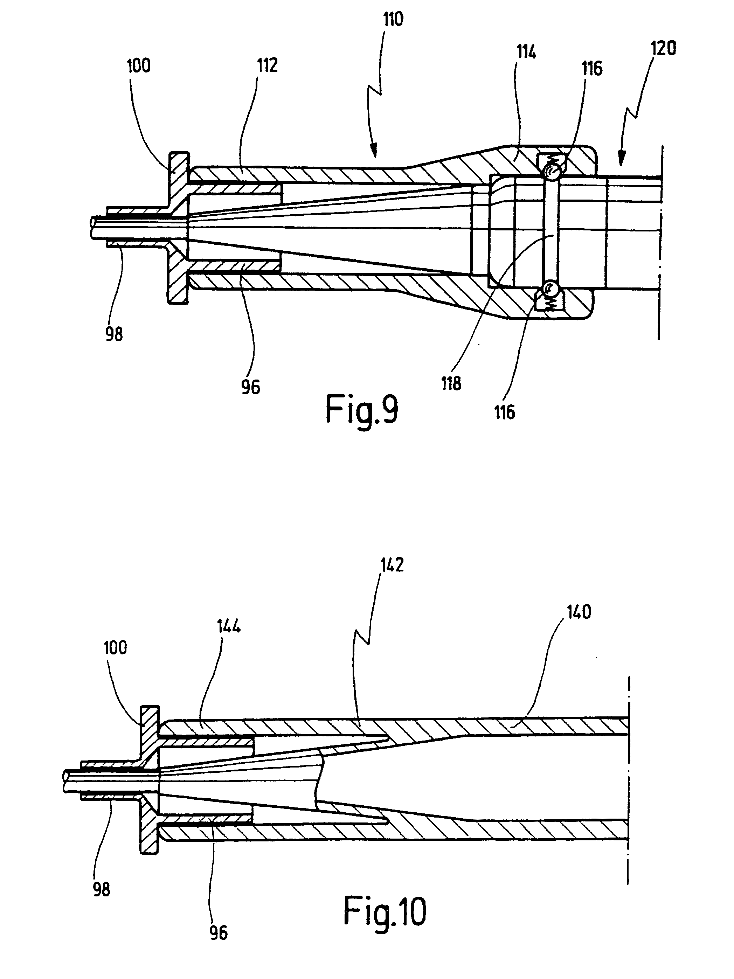

In Fig. 9 ist ein weiteres Ausführungsbeispiel einer erfindungsgemäßen Vorrichtung 110 dargestellt, deren Kupplung 114 zum Verkuppeln mit einem Endoskop 120 einen Kugelrastmechanismus 116 aufweist. Dazu ist an der Außenseite des Endoskopes 120 eine Nut 118 eingeschnitten, in die die entsprechend federbelasteten Kugeln der Kugelraste, die an der Innenseite der Kupplung 114 gehalten sind, einrasten können, wenn die Vorrichtung 110 auf das Endoskop 120 aufgeschoben wird. Am gegenüberliegenden Ende der Kupplung 114 ist eine Aufnahme 112 in Form eines Rohr- bzw. Hohlzapfens vorgesehen, in den, wie zuvor beschrieben, ein proximaler Endabschnitt 96 des Intubationstubus 98 eingeschoben werden kann. Dieser Intubationstubus 98 kann identisch wie der zuvor in Zusammenhang mit Fig. 7 und 8 beschriebene ausgebildet sein, daher wurden auch dieselben Bezugsziffern verwendet.

In Fig. 10 ist eine weitere Ausgestaltung einer erfindungsgemäßen Vorrichtung 144 dargestellt, die fest über Stege 142 mit einem Endoskop 140 verbunden ist. Der Steg 142 stellt somit die Kupplung zum hier festen und unlösbaren Verkuppeln der Vorrichtung 144 mit dem Endoskop 140 dar.

Die Aufnahme für den Intubationstubus 98 wird wieder durch ein rohrförmiges Ende der Vorrichtung 144 ausgebildet, in die der proximale Endabschnitt 96 des Intubationstubus 80 eingeschoben ist. Auch hier können wieder die entsprechenden Flächen leicht konisch ausgebildet sein.

## Patentansprüche

1. Vorrichtung zum Einführen eines Intubationstubus (23; 98) in die Trachea,
mit einem Schaft (12),
mit einer am distalen Ende (14) des Schaftes (12) angeordneten Aufnahme (16; 92; 112), an die ein proximales Ende (25; 96) eines Intubationstubus (23; 98) anbringbar ist, und
mit einer am proximalen Ende (32) des Schaftes (12) angeordneten Kupplung (34; 88; 114) zum Ankoppeln eines Endoskopes (52; 102; 120; 140),
**dadurch gekennzeichnet, daß** die Länge des Schaftes (12) veränderbar ist.

2. Vorrichtung nach Anspruch 1, **dadurch gekennzeichnet, daß** der Schaft (12) teleskopartig aufgebaut ist.

3. Vorrichtung nach Anspruch 1 oder 2, **dadurch gekennzeichnet, daß** der Schaft (12) rohrförmig ist.

4. Vorrichtung nach Anspruch 3, **dadurch gekennzeichnet, daß** der Schaft (12) aus mehreren teleskopartig ineinanderschiebbbaren Rohren (28, 29, 30; 82, 84, 86) aufgebaut ist.

5. Vorrichtung nach einem der Ansprüche 1 bis 4, **dadurch gekennzeichnet, daß** am proximalen Ende (32) ein Gasanschluß (42) angeordnet ist, der mit dem Innenraum des hohlen Schaftes (12) verbunden ist.

6. Vorrichtung nach einem der Ansprüche 1 bis 5, **dadurch gekennzeichnet, daß** am distalen Ende (14) eine Handhabe (24; 94) angeordnet ist, über die die Vorrichtung (10; 110) handhabbar ist.

7. Vorrichtung nach einem der Ansprüche 1 bis 6, **dadurch gekennzeichnet, daß** die Aufnahme (16) als Zapfen (20) ausgebildet ist, auf den ein proximales Ende (25) des Intubationstubus (23) aufschiebbar ist.

8. Vorrichtung nach einem der Ansprüche 1 bis 6, **dadurch gekennzeichnet, daß** die Aufnahme (92; 112) als Hohlzapfen ausgebildet ist, in den ein proximales Ende (96) des Intubationstubus (98) einschiebbar ist.

9. Vorrichtung nach Anspruch 7 oder 8, **dadurch gekennzeichnet, daß** der Zapfen (20) leicht konisch ausgebildet ist.

10. Vorrichtung nach einem der Ansprüche 1 bis 9, **dadurch gekennzeichnet, daß** der Schaft (12) derart ausgebildet ist, daß ein Schaft (58) eines Endoskopes (52) von proximal durch die Vorrichtung (10) hindurchschiebbar ist.

11. Vorrichtung nach Anspruch 10, **dadurch gekennzeichnet, daß** der Schaft (12) derart ausgebildet ist, daß auch bei eingeschobenem Endoskop (52) noch ein Gas durch den Schaft (12) hindurchführbar ist.

12. Vorrichtung nach einem der Ansprüche 1 bis 9, **dadurch gekennzeichnet, daß** diese über deren Länge mit einem Schlitz versehen ist, über den die Vorrichtung an einen Schaft eines Endoskopes anlegbar ist.

13. Vorrichtung nach einem der Ansprüche 1 bis 12, **dadurch gekennzeichnet, daß** die Vorrichtung (80) über die Kupplung (88) an das Endoskop (102) anklemmbar ist.

14. Vorrichtung nach einem der Ansprüche 1 bis 12, **dadurch gekennzeichnet, daß** die Kupplung (114) einen Rastmechanismus (116) zum Verrasten mit dem Endoskop (120) aufweist.

15. Vorrichtung nach Anspruch 14, **dadurch gekennzeichnet, daß** der Rastmechanismus (116) zumindest eine Kugelraste aufweist, und daß am Endoskop (120) zumindest eine Vertiefung ausgebildet ist, in die die Kugelraste eingreift.

16. Vorrichtung nach Anspruch 15, **dadurch gekennzeichnet, daß** die Vertiefung als umfängliche Nut (118) ausgebildet ist.

17. Vorrichtung nach einem der Ansprüche 1 bis 12, **dadurch gekennzeichnet, daß** die Vorrichtung mittels einer Überwurfmutter-Gewinde-Verbindung am Endoskop befestigbar ist.

18. Vorrichtung nach einem der Ansprüche 1 bis 12, **dadurch gekennzeichnet, daß** die Kupplung (34) mittels einer Bajonettverriegelung am Endoskop (52) befestigbar ist.

19. Vorrichtung nach einem der Ansprüche 1 bis 18, **dadurch gekennzeichnet, daß** auf dem distalen Ende (14) ein Intubationstubus (23; 98) angebracht ist, und daß am proximalen Ende (33) ein Endoskop (52; 102; 120) angekoppelt ist.

20. Vorrichtung nach einem der Ansprüche 1 bis 12, **dadurch gekennzeichnet, daß** diese einstückig mit dem Endoskop (140) ausgebildet ist.

21. Vorrichtung nach einem der Ansprüche 1 bis 20, **dadurch gekennzeichnet, daß** das Endoskop starr (52), semi-flexibel oder flexibel (102) ausgebildet ist.

22. Vorrichtung nach Anspruch 21, **dadurch gekennzeichnet, daß** das Endoskop (52) mit einer Kamera versehen ist.

## Claims

1. Device for introducing an intubation tube (23; 98) into the trachea, comprising
a shaft (12),
having a receptor (16; 92; 112), arranged at a distal end (14) of the shaft (12), to which a proximal end (25; 96) of an intubation tube (23; 98) can be attached, and
a coupling (34; 88; 114) is arranged at the proximal end (32) of the shaft (12) for coupling an endoscope (52; 102; 120; 140) thereon,
**characterized in that** the length of the shaft (12) is variable.

2. Device of claim 1, **characterized in that** the shaft (12) is configured to be telescopic.

3. Device of claim 1 or 2, **characterized in that** the shaft (12) is tubular.

4. Device of claim 3, **characterized in that** the shaft (12) is configured of several telescopic tubes (28, 29, 30; 82, 84, 86) shiftable within one another.

5. Device of anyone of claims 1 through 4, **characterized in that** a gas connector (42) is arranged at the proximal end (32), which communicates with the interior of the hollow shaft (12).

6. Device of anyone of claims 1 through 5, **characterized in that** a handle (24; 94) is arranged at the distal end (14), through which the device (10; 110) can be handled.

7. Device of anyone of claims 1 through 6, **characterized in that** the receptor (16) is configured as a stud (20), onto which a proximal end (25) of the intubation tube (23) can be slipped.

8. Device of anyone of claims 1 through 6, **characterized in that** the receptor (92; 112) is configured as a hollow stud, into which a proximal end (96) of the intubation tube (98) can be inserted.

9. Device of claims 7 or 8, **characterized in that** the stud (20) is configured to be slightly conical.

10. Device of anyone of claims 1 through 9, **characterized in that** the shaft (12) is configured such that a shaft (58) of an endoscope (52) can be passed through the device (10) from the proximal end.

11. Device of claim 10, **characterized in that** the shaft (12) is configured such that a gas can be passed through the shaft (12) also when an endoscope (52) is inserted.

12. Device of anyone of claims 1 through 9, **characterized in that** a slot is provided over its length, via which the device can be placed onto a shaft of an endoscope.

13. Device of anyone of claims 1 through 12, **characterized in that** the device (80) can be clamped to the endoscope (102) by the coupling (88).

14. Device of anyone of claims 1 through 12, **characterized in that** the coupling (114) comprises a locking mechanism (116) for locking to the endoscope (120).

15. Device of claim 14, **characterized in that** the locking mechanism (116) comprises at least one ball catch, and **in that** the endoscope (120) has at least one recess into which the ball catch engages.

16. Device of claim 15, **characterized in that** the recess is formed as a circumferential groove (118).

17. Device of anyone of claims 1 through 12, **characterized in that** the device can be secured to the endoscope through a union nut-thread-connection.

18. Device of anyone of claims 1 through 12, **characterized in that** the coupling (34) can be secured to the endoscope (52) by a bayonet locking.

19. Device of anyone of claims 1 through 18, **characterized in that** an intubation tube (23, 98) is attached to the distal end (14), and **in that** an endoscope (52; 102; 120) is coupled to the proximal end (33).

20. Device of anyone of claims 1 through 12, **characterized in that** it is integrally formed with the endoscope (140).

21. Device of anyone of claims 1 through 20, **characterized in that** the endoscope is configured to be rigid (52), semi-flexible or flexible (102).

22. Device of claim 21, **characterized in that** the endoscope (52) is provided with a camera.

## Revendications

1. Dispositif d'introduction d'un tube d'intubation (23 ; 98) dans la trachée,
avec une tige (12),
avec un récepteur (16 ; 92 ; 112) placé à l'extrémité distale (14) de la tige (12), sur lequel peut être rapportée une extrémité proximale (25 ; 96) d'un tube d'intubation (23 ; 98), et
avec un accouplement (34 ; 88 ; 114) placé à l'extrémité proximale (32) de la tige (12) pour coupler un endoscope (52 ; 102 ; 120 ; 140),
**caractérisé en ce que** la longueur de la tige (12) est variable.

2. Dispositif selon la revendication 1, **caractérisé en ce que** la tige (12) est de construction télescopique.

3. Dispositif selon la revendication 1 ou 2, **caractérisé en ce que** la tige (12) est tubulaire.

4. Dispositif selon la revendication 3, **caractérisé en ce que** la tige (12) est composée de plusieurs tubes (28, 29, 30 ; 82, 84, 86) escamotables de façon télescopique les uns dans les autres.

5. Dispositif selon l'une des revendications 1 à 4, **caractérisé en ce qu'**à l'extrémité proximale (32) est placé un raccord de gaz (42) qui communique avec l'intérieur de la tige creuse (12).

6. Dispositif selon l'une des revendications 1 à 5, **caractérisé en ce qu'**à l'extrémité distale (14) est placée une poignée (24 ; 94) par l'intermédiaire de laquelle le dispositif (10 ; 110) peut être tenu.

7. Dispositif selon l'une des revendications 1 à 6, **caractérisé en ce que** le récepteur (16) est conformé en tenon (20) sur lequel une extrémité proximale (25) du tube d'intubation (23).

8. Dispositif selon l'une des revendications 1 à 6, **caractérisé en ce que** le récepteur (92 ; 112) est conformé en tenon creux dans lequel une extrémité proximale (96) du tube d'intubation (98) peut être enfilée.

9. Dispositif selon la revendication 7 ou 8, **caractérisé en ce que** le tenon (20) est réalisé légèrement conique.

10. Dispositif selon l'une des revendications 1 à 9, **caractérisé** en ce la tige (12) est conformée de telle manière qu'une tige (58) d'un endoscope (52) peut être enfilée à travers le dispositif (10) depuis le côté proximal.

11. Dispositif selon la revendication 1à, **caractérisé en ce que** la tige (12) est conformée de telle manière que même avec l'endoscope (52) enfoncé, un gaz peut encore être introduit à travers la tige (12).

12. Dispositif selon l'une des revendications 1 à 9, **caractérisé en ce qu'**il est muni sur sa longueur d'une fente par l'intermédiaire de laquelle le dispositif peut être rapporté sur une tige d'un endoscope.

13. Dispositif selon l'une des revendications 1 à 12, **caractérisé en ce que** le dispositif (10) peut être fixé sur l'endoscope (102) par l'intermédiaire de l'accouplement (88).

14. Dispositif selon l'une des revendications 1 à 12, **caractérisé en ce que** l'accouplement (114) présente un mécanisme d'encliquetage (116) pour s'encliqueter avec l'endoscope (120).

15. Dispositif selon la revendication 14, **caractérisé en ce que** le mécanisme d'encliquetage (116) présente au moins un cliquet à bille, et **en ce qu'**au moins un renfoncement est formé sur l'endoscope (120), dans lequel s'engage le cliquet à bille.

16. Dispositif selon la revendication 15, **caractérisé en ce que** le renfoncement est conformé en rainure périphérique (118).

17. Dispositif selon l'une des revendications 1 à 12, **caractérisé en ce que** le dispositif peut être fixé sur l'endoscope au moyen d'une liaison écrou d'accouplement-filetage.

18. Dispositif selon l'une des revendications 1 à 12, **caractérisé en ce que** l'accouplement (34) peut être fixé à l'endoscope (52) au moyen d'un verrouillage à baïonnette.

19. Dispositif selon l'une des revendications 1 à 18, **caractérisé en ce qu'**à l'extrémité distale (14) est placé un tube d'intubation (23 ; 98), et **en ce qu'**à l'extrémité proximale (33) est accouplé un endoscope (52 ; 102 ; 120).

20. Dispositif selon l'une des revendications 1 à 12, **caractérisé en ce qu'**il est réalisé d'un seul tenant avec l'endoscope (140).

21. Dispositif selon l'une des revendications 1 à 20, **caractérisé en ce que** l'endoscope est réalisé rigide (52), semi-flexible ou flexible (102).

22. Dispositif selon la revendication 21, **caractérisé en ce que** l'endoscope (52) est muni d'une caméra.
